# EUROPEAN PATENT APPLICATION

(11) **EP 3 045 116 A1**
(43) Date of publication of application: **20.07.2016**
(21) Application number: 15175446.2
(22) Date of filing: 06.07.2015
(51) Int. Cl.: A61B 8/06, A61B 8/00, A61B 8/08, A61B 8/02

(54) **ULTRASOUND DIAGNOSIS APPARATUS AND METHOD OF OPERATING THE SAME**

(30) Priority: 13.01.2015 KR 20150006112
(71) Applicant: Samsung Medison Co., Ltd., Gangwon-do 250-870 (KR)
(72) Inventor: Kim, Ji-han, Gangwon-do (KR); Lee, Jae-keun, Gangwon-do (KR)
(74) Representative: Frey, Sven Holger

(57) **Abstract**

Provided is an ultrasound diagnosis apparatus including: a data acquisition unit configured to acquire color Doppler data from a region of interest (ROI) of an object; a blood flow information extractor configured to analyze a change in blood flow with respect to time based on the acquired color Doppler data and extract blood flow information based on the change in blood flow; and a display configured to display the extracted blood flow information.

## Description

### RELATED APPLICATION

This application claims the benefit of Korean Patent Application No. 10-2015-0006112, filed on January 13, 2015, in the Korean Intellectual Property Office, the disclosure of which is incorporated herein in its entirety by reference.

### BACKGROUND

### 1. Field

One or more exemplary embodiments relate to an ultrasound diagnosis apparatus and a method of operating the same, and more particularly, to an ultrasound diagnosis apparatus and method of operating the same which are capable of extracting information based on a color flow image.

### 2. Description of the Related Art

Ultrasound diagnosis apparatuses transmit ultrasound signals generated by transducers of a probe to an object and receive echo signals reflected from the object, thereby obtaining at least one image of an internal part of the object (e.g., soft tissue or blood flow). In particular, ultrasound diagnosis apparatuses are used for medical purposes including observation of the interior of an object, detection of foreign substances, and diagnosis of damage to the object. Such ultrasound diagnosis apparatuses provide high stability, display images in real time, and are safe due to no radiation exposure, compared to X-ray apparatuses. Therefore, ultrasound diagnosis apparatuses are widely used together with other image diagnosis apparatuses including a computed tomography (CT) apparatus, a magnetic resonance imaging (MRI) apparatus, and the like.

An ultrasound diagnosis apparatus may support a brightness (B) mode, a Doppler mode, an elastic mode, and the like. In the B mode, a reflection coefficient of an ultrasound signal is visualized as a two-dimensional (2D) image. In the Doppler mode, a velocity of a moving object (in particular, blood flow) is shown as an image by using the Doppler effect. In the elastic mode, a difference between responses when compression is or not applied to an object is visualized as an image.

### SUMMARY

One or more exemplary embodiments include an ultrasound diagnosis apparatus and a method of operating the same, which are capable of analyzing a change in blood flow based on color Doppler data and extracting blood flow information according to the analyzed change in blood flow.

Additional aspects will be set forth in part in the description which follows and, in part, will be apparent from the description, or may be learned by practice of the presented exemplary embodiments.

According to one or more exemplary embodiments, an ultrasound diagnosis apparatus includes: a data acquisition unit configured to acquire color Doppler data from a region of interest (ROI) of an object; a blood flow information extractor configured to analyze a change in blood flow with respect to time based on the acquired color Doppler data and extract blood flow information based on the change in blood flow; and a display configured to display the extracted blood flow information.

The color Doppler data may include at least one of power data of blood flow through the ROI, velocity data of the blood flow, variance data of the blood flow, and blood flow amount data.

The blood flow information may include at least one of a cardiac cycle duration, a heart rate, and a heartbeat landmark point for the object.

The heartbeat landmark point may include a peak point appearing as a heart contracts and relaxes.

The blood flow information extractor may generate a waveform showing the change in blood flow with respect to time and extract the blood flow information based on the waveform.

The display may display a graph showing the change in blood flow with respect to time.

The blood flow information extractor may set a parameter corresponding to the ROI based on the change in blood flow and extract the blood flow information based on a change in the parameter.

The parameter may include at least one of a power threshold, a power ceiling, a velocity threshold, a velocity ceiling, a scale, and a baseline.

The blood flow information extractor may set the parameter by generating a histogram based on the color Doppler data and analyzing the histogram.

The blood flow information extractor may set the parameter by analyzing a spatial distribution of the color Doppler data.

The blood flow information extractor may set a power threshold to increase as a value of power data of blood flow through the ROI increases and to decrease as the value of the power data decreases.

The ultrasound diagnosis apparatus may further include an image generator configured to generate a color flow image of the ROI, and the display may display the color flow image.

According to one or more exemplary embodiments, a method of operating an ultrasound diagnosis apparatus includes: acquiring color Doppler data from an ROI of an object; analyzing a change in blood flow with respect to time based on the acquired color Doppler data and extracting blood flow information based on the change in blood flow; and displaying the extracted blood flow information.

The color Doppler data may include at least one of power data of blood flow through the ROI, velocity data of the blood flow, variance data of the blood flow, and blood flow amount data.

The blood flow information may include at least one of a cardiac cycle duration, a heart rate, and a heartbeat landmark point for the object.

The heartbeat landmark point may include a peak point appearing as a heart contracts and relaxes.

The extracting of the blood flow information may include generating a waveform showing the change in blood flow with respect to the time and extracting the blood flow information based on the waveform.

The displaying of the blood flow information may include displaying a graph showing the change in blood flow with respect to the time.

The extracting of the blood flow information may include setting a parameter corresponding to the ROI based on the change in blood flow and extracting the blood flow information based on a change in the parameter.

The parameter may include at least one of a power threshold, a power ceiling, a velocity threshold, a velocity ceiling, a scale, and a baseline.

The extracting of the blood flow information may include setting the parameter by generating a histogram based on the color Doppler data and analyzing the histogram.

The extracting of the blood flow information may include setting the parameter by analyzing a spatial distribution of the color Doppler data.

The extracting of the blood flow information may include setting a power threshold to increase as a value of power data of blood flow through the ROI increases and to decrease as the value of the power data decreases.

The method may further include generating a color flow image of the ROI and displaying the color flow image.

The generating of the color flow image may include generating the color flow image by using power data that has a value that is greater than or equal to a set power threshold among power data of blood flow through the ROI.

According to the exemplary embodiments, in order to obtain blood flow information of an ROI, the blood flow information may be extracted using flow data acquired to generate a color flow image instead of having to acquire a separate spectral Doppler image. Thus, it is possible to reduce operations performed by a user and increase scanning efficiency and improve convenience of use.

Furthermore, to obtain blood flow information, a separate sample volume does not need to be set, and separate hardware is not required.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 is a block diagram of a configuration of an ultrasound diagnosis apparatus according to an exemplary embodiment.
FIG. 2 is a block diagram of a configuration of an ultrasound diagnosis apparatus according to another exemplary embodiment.
FIGS. 3 and 4 are reference diagrams for explaining a method of setting a parameter according to a change in blood flow according to an exemplary embodiment;
FIG. 5 illustrates graphs of blood flow power with respect to time and a threshold for the blood flow power with respect to time, according to an exemplary embodiment;
FIG. 6 is a reference diagram for explaining a method of extracting blood flow information according to an exemplary embodiment;
FIG. 7 is an example where a color flow image and extracted blood flow information are displayed on a display, according to an exemplary embodiment;
FIG. 8A is an example where an ultrasound image and a spectral Doppler image are displayed on a display, and FIG. 8B is an example where an ultrasound image and blood flow information according to an exemplary embodiment are displayed on a display; and
FIG. 9 is a flowchart of a method of operating an ultrasound diagnosis apparatus according to an exemplary embodiment.

### DETAILED DESCRIPTION

Reference will now be made in detail to exemplary embodiments, examples of which are illustrated in the accompanying drawings, wherein like reference numerals refer to like structural elements throughout. In this regard, the present exemplary embodiments may have different forms and should not be construed as being limited to the descriptions set forth herein. Accordingly, the exemplary embodiments are merely described below, by referring to the figures, to explain aspects of the present description. Expressions such as "at least one of," when preceding a list of elements, modify the entire list of elements and do not modify the individual elements of the list.

The terms used in this specification are those general terms currently widely used in the art in consideration of functions regarding the inventive concept, but the terms may vary according to the intention of those of ordinary skill in the art, precedents, or new technology in the art. Also, some terms may be arbitrarily selected by the applicant, and in this case, the meaning of the selected terms will be described in detail in the detailed description of the invention. Thus, the terms used in the specification should be understood not as simple names but based on the meaning of the terms and the overall description of the invention.

Throughout the specification, it will also be understood that when a component "includes" an element, unless there is another opposite description thereto, it should be understood that the component does not exclude another element and may further include another element. In addition, terms such as "... unit", "... module", or the like refer to units that perform at least one function or operation, and the units may be implemented as hardware or software or as a combination of hardware and software.

Throughout the specification, an "ultrasound image" refers to an image of an object, which is obtained using ultrasound waves. Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom means a material having a density, an effective atomic number, and a volume that are approximately the same as those of an organism. For example, the phantom may be a spherical phantom having properties similar to a human body.

Furthermore, an ultrasound image may take different forms. For example, the ultrasound image may be at least one selected from an amplitude (A) mode image, a brightness (B) mode image, a color (C) mode image, and a Doppler (D) mode image. In addition, according to an exemplary embodiment, the ultrasound image may be a two-dimensional (2D) or three-dimensional (3D) image.

Throughout the specification, a "user" may be, but is not limited to, a medical expert, for example, a medical doctor, a nurse, a medical laboratory technologist, or a medical imaging expert, or a technician who repairs medical apparatuses.

Reference will now be made in detail to embodiments, examples of which are illustrated in the accompanying drawings. In this regard, the present embodiments may have different forms and should not be construed as being limited to the descriptions set forth herein.

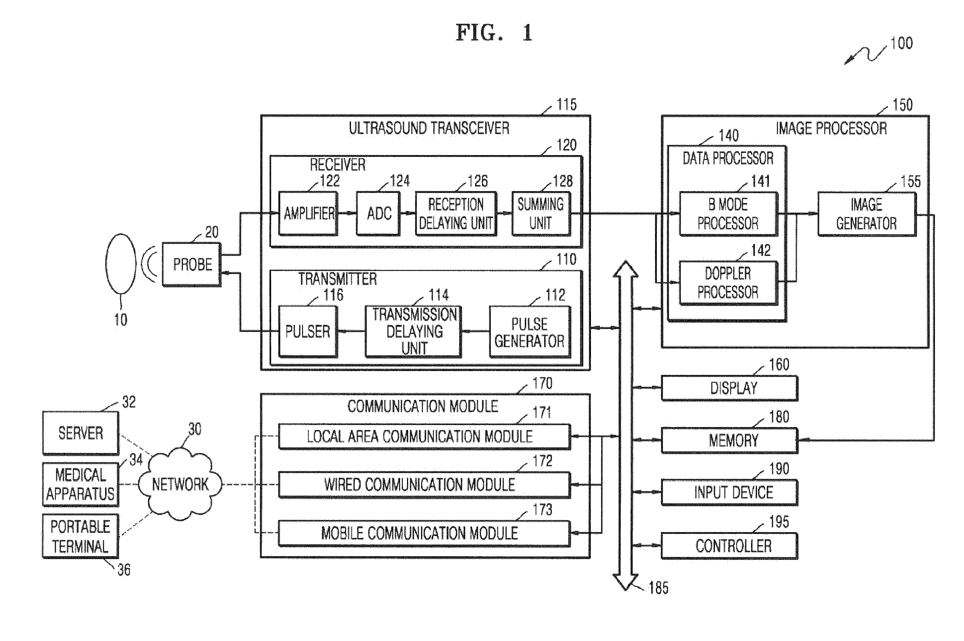

FIG. 1 is a block diagram showing a configuration of an ultrasound diagnosis apparatus 100 according to an embodiment. Referring to FIG. 1, the ultrasound diagnosis apparatus 100 may include a probe 20, an ultrasound transceiver 115, an image processor 150, a communication module 170, a display 160, a memory 180, an input device 190, and a controller 195, which may be connected to one another via buses 185.

In some embodiments, the ultrasound diagnosis apparatus 100 may be a cart type apparatus or a portable type apparatus. Examples of portable ultrasound diagnosis apparatuses may include, but are not limited to, a picture archiving and communication system (PACS) viewer, a smartphone, a laptop computer, a personal digital assistant (PDA), and a tablet PC.

The probe 20 transmits ultrasound waves to an object 10 in response to a driving signal applied by the ultrasound transceiver 115 and receives echo signals reflected by the object 10. The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis apparatus 100 by wire or wirelessly, and according to embodiments, the ultrasound diagnosis apparatus 100 may include a plurality of probes 20.

A transmitter 110 supplies a driving signal to the probe 20. The transmitter 110 includes a pulse generator 112, a transmission delaying unit 114, and a pulser 116. The pulse generator 112 generates pulses for forming transmission ultrasound waves based on a predetermined pulse repetition frequency (PRF), and the transmission delaying unit 114 delays the pulses by delay times necessary for determining transmission directionality. The pulses which have been delayed correspond to a plurality of piezoelectric vibrators included in the probe 20, respectively. The pulser 116 applies a driving signal (or a driving pulse) to the probe 20 based on timing corresponding to each of the pulses which have been delayed.

A receiver 120 generates ultrasound data by processing echo signals received from the probe 20. The receiver 120 may include an amplifier 122, an analog-to-digital converter (ADC) 124, a reception delaying unit 126, and a summing unit 128. The amplifier 122 amplifies echo signals in each channel, and the ADC 124 performs analog-to-digital conversion with respect to the amplified echo signals. The reception delaying unit 126 delays digital echo signals output by the ADC 124 by delay times necessary for determining reception directionality, and the summing unit 128 generates ultrasound data by summing the echo signals processed by the reception delaying unit 126. In some embodiments, the receiver 1120 may not include the amplifier 122. In other words, if the sensitivity of the probe 20 or the capability of the ADC 124 to process bits is enhanced, the amplifier 122 may be omitted.

The image processor 150 generates an ultrasound image by scan-converting ultrasound data generated by the ultrasound transceiver 115 and displays the ultrasound image. The ultrasound image may be not only a grayscale ultrasound image obtained by scanning an object in an amplitude (A) mode, a brightness (B) mode, and a motion (M) mode, but also a Doppler image showing a movement of an object via a Doppler effect. The Doppler image may be a blood flow Doppler image showing flow of blood (also referred to as a color flow image), a tissue Doppler image showing a movement of tissue, or a spectral Doppler image showing a moving speed of an object as a waveform.

A B mode processor 141 extracts B mode components from ultrasound data and processes the B mode components. An image generator 155 may generate an ultrasound image indicating signal intensities as brightness based on the extracted B mode components 141.

Similarly, a Doppler processor 142 may extract Doppler components from ultrasound data, and the image generator 155 may generate a Doppler image (e.g., a color flow image, etc.) indicating a movement of an object as colors or waveforms based on the extracted Doppler components.

According to an exemplary embodiment, the Doppler processor 142 may analyze a change in blood flow based on color Doppler data being acquired in real-time among ultrasound data and set parameters according to the change in blood flow. The color Doppler data may include at least one selected from power data representing blood flow power, velocity data representing a blood flow velocity, and variance data representing blood flow variance.

Furthermore, to generate a color flow image, the parameters may include a parameter that is applied to color Doppler data. For example, the parameter may include a power threshold or power ceiling that is applied to blood flow power data, a velocity threshold or velocity ceiling that is applied to blood flow velocity data, a scale of a color flow image, and a baseline.

The Doppler processor 142 may analyze blood flow power data to set a power threshold or power ceiling that is applied to the blood flow power data. The Doppler processor 142 may also analyze blood flow velocity data to set a velocity threshold or velocity ceiling that is applied to the blood flow velocity data. However, exemplary embodiments are not limited thereto, and the Doppler processor 142 may set parameters necessary for generating a color flow image based on at least one selected from blood flow power data, blood flow velocity data, and blood flow variance data.

According to an embodiment, the image generator 155 may generate a three-dimensional (3D) ultrasound image via volume-rendering with respect to volume data and may also generate an elasticity image by imaging deformation of the object 10 due to pressure.

The image generator 155 may also generate a color flow image by applying parameters set by the Doppler processor 142 to color Doppler data. The color flow image may show information about movement of an object such as blood flow in a color.

Furthermore, the image generator 155 may display various pieces of additional information in an ultrasound image by using text and graphics. In addition, the generated ultrasound image may be stored in the memory 180.

A display 160 displays the generated ultrasound image. The display 160 may display not only an ultrasound image, but also various pieces of information processed by the ultrasound diagnosis apparatus 100 on a screen image via a graphical user interface (GUI). In addition, the ultrasound diagnosis apparatus 100 may include two or more displays 160 according to embodiments.

The display 160 may include at least one of a liquid crystal display (LCD), a thin film transistor-LCD (TFT-LCD), an organic light-emitting diode (OLED) display, a flexible display, a 3D display, and an electrophoretic display.

Furthermore, when the display 160 and a the input device 190 form a layer structure to form a touch screen, the display 160 may be used as an input device as well as an output device, via which a user inputs information via a touch.

The touch screen may be configured to detect a position of a touch input, a touched area, and pressure of a touch. The touch screen may also be configured to detect both a real touch and a proximity touch.

In the present specification, a 'real touch' means that a pointer actually touches a screen, and a 'proximity touch' means that a pointer does not actually touch a screen but approaches the screen while being separated from the screen by a predetermined distance. A 'pointer' used herein means a tool for touching a particular portion on or near a displayed screen. Examples of the pointer may include a stylus pen and a body part such as fingers.

Although not shown, the ultrasound diagnosis apparatus 100 may include various sensors that are disposed within or near the touch screen so as to sense a real touch or proximity touch on the touch screen. A tactile sensor is an example of the sensors for sensing a touch on the touch screen.

The tactile sensor is used to sense a touch of a particular object to the same or greater degree than the degree to which a human can sense the touch. The tactile sensor may detect various pieces of information including the roughness of a contact surface, the hardness of an object to be touched, the temperature of a point to be touched, etc.

A proximity sensor is another example of the sensors for sensing a touch. The proximity sensor refers to a sensor that senses the presence of an object that is approaching or is located near a predetermined detection surface by using the force of an electromagnetic field or infrared light without mechanical contact

Examples of the proximity sensor include a transmissive photoelectric sensor, a direct reflective photoelectric sensor, a mirror reflective photoelectric sensor, a highfrequency oscillation proximity sensor, a capacitive proximity sensor, a magnetic proximity sensor, an infrared proximity sensor, and the like.

The communication module 170 is connected to a network 30 by wire or wirelessly to communicate with an external device or a server. The communication module 170 may exchange data with a hospital server or another medical apparatus in a hospital, which is connected thereto via a PACS. Furthermore, the communication module 170 may perform data communication according to the digital imaging and communications in medicine (DICOM) standard.

The communication module 170 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 170 may receive information about a diagnosis history or medical treatment schedule of a patient from a server and utilizes the received information to diagnose the patient. Furthermore, the communication module 170 may perform data communication not only with a server or a medical apparatus in a hospital, but also with a portable terminal of a medical doctor or patient.

The communication module 170 is connected to the network 30 by wire or wirelessly to exchange data with a server 32, a medical apparatus 34, or a portable terminal 36. The communication module 170 may include one or more components for communication with external devices. For example, the communication module 1300 may include a local area communication module 171, a wired communication module 172, and a mobile communication module 173.

The local area communication module 171 refers to a module for local area communication within a predetermined distance. Examples of local area communication techniques according to an embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 172 refers to a module for communication using electric signals or optical signals. Examples of wired communication techniques according to an embodiment may include communication via a twisted pair cable, a coaxial cable, an optical fiber cable, and an Ethernet cable.

The mobile communication module 173 transmits or receives wireless signals to or from at least one selected from a base station, an external terminal, and a server on a mobile communication network. The wireless signals may be voice call signals, video call signals, or various types of data for transmission and reception of text/multimedia messages.

The memory 180 stores various data processed by the ultrasound diagnosis apparatus 100. For example, the memory 180 may store medical data related to diagnosis of an object, such as ultrasound data and an ultrasound image that are input or output, and may also store algorithms or programs which are to be executed in the ultrasound diagnosis apparatus 100.

The memory 180 may be any of various storage media, e.g., a flash memory, a hard disk drive, EEPROM, etc. Furthermore, the ultrasound diagnosis apparatus 100 may utilize web storage or a cloud server that performs the storage function of the memory 180 online.

The input device 190 refers to a means via which a user inputs data for controlling the ultrasound diagnosis apparatus 100. The input device 190 may include hardware components, such as a keypad, a mouse, a touch pad, a track ball, and a jog switch. However, embodiments are not limited thereto, and the input device 1600 may further include any of various other input units including an electrocardiogram (ECG) measuring module, a respiration measuring module, a voice recognition sensor, a gesture recognition sensor, a fingerprint recognition sensor, an iris recognition sensor, a depth sensor, a distance sensor, etc. In particular, the input device 190 may also include a touch screen in which a touch pad forms a layer structure with the display 160.

In this case, according to an exemplary embodiment, the ultrasound diagnosis apparatus 100 may display an ultrasound image in a predetermined mode and a control panel for the ultrasound image on a touch screen. The ultrasound diagnosis apparatus 100 may also sense a user's touch gesture performed on an ultrasound image via a touch screen.

The ultrasound diagnosis apparatus 100 may include some buttons that are frequently used by a user among buttons that are included in a control panel of a general ultrasound apparatus, and provide the remaining buttons in the form of a GUI via a touch screen.

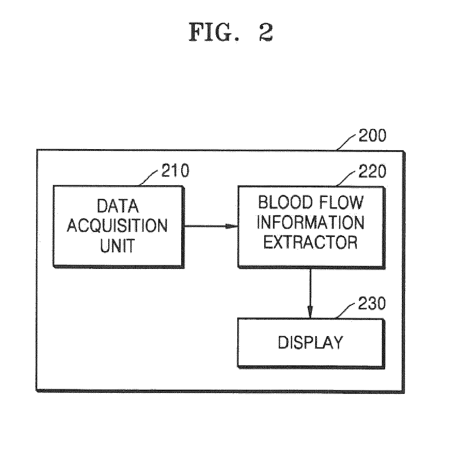

The controller 195 may control all operations of the ultrasound diagnosis apparatus 100. In other words, the controller 195 may control operations among the probe 20, the ultrasound transceiver 100, the image processor 150, the communication module 170, the memory 180, and the input device 190 shown in FIG. 2.

All or some of the probe 20, the ultrasound transceiver 115, the image processor 150, the display 160, the communication module170, the memory 180, the input device 190, and the controller 195 may be implemented as software modules. Furthermore, at least one selected from the ultrasound transceiver 115, the image processor 150, and the communication module 170 may be included in the controller 195. However, embodiments of the present invention are not limited thereto.

FIG. 2 is a block diagram of a configuration of an ultrasound diagnosis apparatus 200 according to another exemplary embodiment. Referring to FIG. 2, the ultrasound diagnosis apparatus 200 according to the present exemplary embodiment may include a data acquisition unit 210, a blood flow information extractor 220, and a display 230.

The data acquisition unit 210 shown in FIG. 2 may correspond to the probe 20 or the ultrasound transceiver 115 shown in FIG. 1. The blood flow information extractor 220 and the display 230 shown in FIG. 2 may respectively correspond to the data processor 140 and the display 160 shown in FIG. 1.

The data acquisition unit 210 may acquire color Doppler data for a region of interest (ROI). For example, if the ROI is a blood vessel, the data acquisition unit 210 may acquire at least one selected from power data representing power of blood flow through the blood vessel, velocity data representing a velocity of the blood flow, and variance data representing a variance of the blood flow.

The data acquisition unit 210 may acquire color Doppler data for an ROI by transmitting an ultrasound signal to the ROI and receiving an echo signal reflected from the ROI. Alternatively, the data acquisition unit 210 may receive color Doppler data from an external device. In this case, the data acquisition unit 210 may receive the color Doppler data from the external device via the communication module 170 described with reference to FIG. 1.

The blood flow information extractor 220 may analyze a change in blood flow through an ROI based on color Doppler data acquired in real-time and then extract blood flow information according to the change in blood flow.

For example, the change in blood flow may include changes in blood flow amount (power), blood flow velocity, and blood flow distribution. Furthermore, the blood flow information may include at least one of a cardiac cycle duration, a heart rate, and a heartbeat landmark point for an object. The heartbeat landmark point may include a peak point appearing as a heart muscle contracts and relaxes.

For example, the blood flow information extractor 220 may generate a waveform showing a change in blood flow over time and extract blood flow information based on the generated waveform. A period of the waveform may represent a cardiac cycle duration. Thus, the blood flow information extractor 220 may extract a peak point (e.g., a heartbeat landmark point) appearing in the waveform and calculate a time interval between adjacent peak points, thereby obtaining a cardiac cycle duration. The blood flow information extractor 220 may also calculate a heart rate based on the acquired cardiac cycle duration. The heart rate may be defined as the reciprocal of the cardiac cycle duration.

Furthermore, the blood flow information extractor 220 may set parameters corresponding to an ROI according to a change in blood flow and extract blood flow information based on a change in parameters.

For example, the blood flow information extractor 220 may analyze a change in blood flow based on color Doppler data acquired in real-time and set parameters corresponding to an ROI according to the change in blood flow. To generate a color flow image of an ROI, the parameters may include a parameter that is applied to color Doppler data. For example, the parameter that is applied to color Doppler data may include a power threshold or power ceiling that is applied to blood flow power data, a velocity threshold or velocity ceiling that is applied to blood flow velocity data, a scale of a color flow image, and a baseline.

In this case, a power threshold and a power ceiling may be parameters for determining a lower limit and an upper limit for power data, respectively. Similarly, a velocity threshold and a velocity ceiling may be parameters for determining a lower limit and an upper limit for velocity data, respectively. A scale of a color flow image may be a parameter for determining a range of values indicated on the color flow image. A baseline may be a parameter for determining a reference value for values indicated on a color flow image.

The blood flow information extractor 220 may analyze power data of blood flow acquired in real-time to set a power threshold or power ceiling that is applied to the power data. Furthermore, the blood flow information extractor 220 may analyze velocity data of blood flow acquired in real-time to set a velocity threshold or velocity ceiling that is applied to the velocity data. However, exemplary embodiments are not limited thereto, and the blood flow information extractor 220 may set parameters necessary for generating a color flow image, based on at least one selected from power data, velocity data, and variance data of blood flow.

The blood flow information extractor 220 may generate a waveform showing a change in blood flow based on a change in a value of a parameter that is set according to time and extract blood flow information based on the generated waveform. Since a method of extracting blood flow information based on the waveform has been described in detail above, a detail description thereof is omitted.

The display 230 may display extracted blood flow information. For example, the display 230 may display a waveform showing a change in blood flow and a heartbeat landmark point on the waveform. The display 230 may also display calculated cardiac cycle duration and heart rate.

In addition, the display 230 may display a color flow image of an ROI together with blood flow information. For example, the image generator 155 shown in FIG. 1 may generate a color flow image by using power data that has a value that is greater than or equal to a set power threshold or less than a set power ceiling among power data of blood flow through the ROI. Alternatively, the image generator 230 may generate a color flow image by using velocity data that has a value that is greater than or equal to a set velocity threshold or less than a set velocity ceiling among velocity data of blood flow corresponding to the ROI. The display 230 may display the generated color flow image.

Furthermore, the display 230 may superimpose a B-mode image of an object and a color flow image of a region of the object set as the ROI on each other for display thereof. However, exemplary embodiments are not limited thereto.

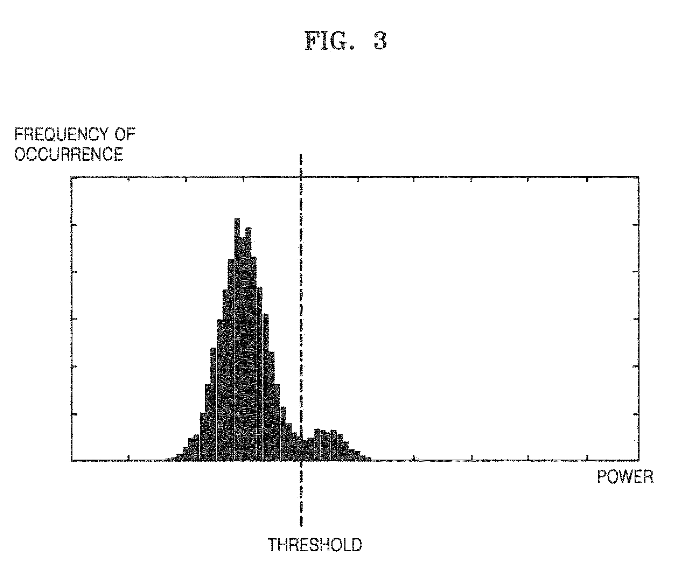

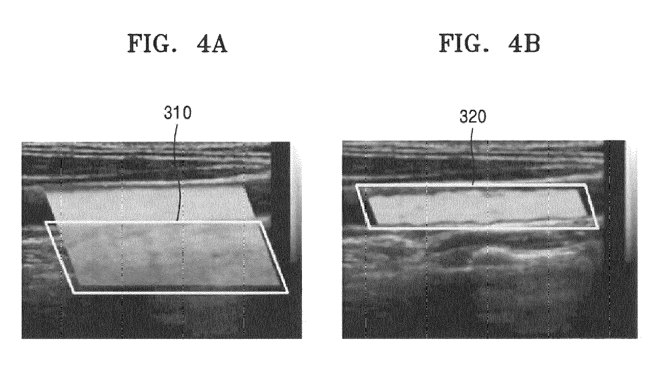

FIGS. 3 and 4 are reference diagrams for explaining a method of setting a parameter according to a change in blood flow according to an exemplary embodiment.

FIG. 3 illustrates a histogram for power data of blood flow corresponding to an ROI. As shown in FIG. 3, the ultrasound diagnosis apparatus 100 of FIG. 1 (200 of FIG. 2) may acquire power data of blood flow corresponding to the ROI and generate the histogram for the acquired power data. The abscissa and ordinate of the histogram respectively indicate power values of blood flow and the frequency of occurrence for each power value.

The blood flow information extractor 220 may extract a power threshold by analyzing a histogram for power data. For example, the blood flow information extractor 220 may extract a power threshold by analyzing the spatial distribution of power data. As shown in FIG. 4A, other than a blood vessel, the blood flow information extractor 220 may set a power threshold value by analyzing power data corresponding to a region 310 outside the blood vessel.

Thus, as shown in FIG. 3, the blood flow information extractor 220 may set a first power value as a power threshold and distinguish noise from an effective signal based on the first power value (power threshold). For example, the ultrasound diagnosis apparatus 100 (200) may determine power values that are less than the first power value as noise, and power values that are greater than or equal to the first power value as effective signals. However, exemplary embodiments are not limited thereto.

FIG. 4A illustrates a color flow image that is generated using power data of blood flow acquired by the data acquisition unit 210. In this case, the color flow image shown in FIG. 4A may be generated using power data acquired without setting a threshold as described with reference to FIG. 3.

On the other hand, FIG. 4B illustrates a color flow image generated based on power data (e.g., an effective signal excluding noise) that has a value that is greater than or equal to a power threshold among power values. As seen on FIGS. 4A and 4B, the color flow image of FIG. 4A depicts blood flow not only in a blood vessel but also in the region 310 outside the blood vessel, whereas the color flow image of FIG. 4B indicates blood flow only in a region 320 within a blood vessel.

Although it has been described with reference to FIGS. 3 and 4 that a color flow image is generated by setting a parameter for power data of blood flow, exemplary embodiments are not limited thereto. The above-described method may also be applied in the same way to blood flow velocity data, blood flow variance data, and the like.

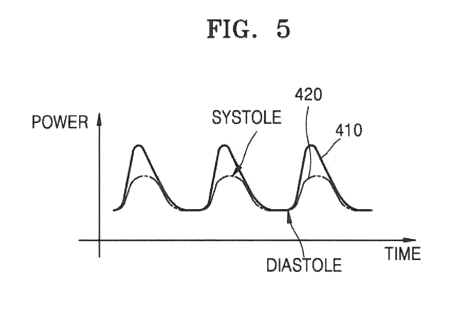

FIG. 5 illustrates graphs of blood flow power with respect to time and a threshold for the blood flow power with respect to time, according to an exemplary embodiment

Referring to FIG. 5, the blood flow information extractor 220 may set a threshold for blood flow power at different values based on power data of blood flow acquired over time. In this case, power data of blood flow may represent the amount of blood flow through a blood vessel. Furthermore, a waveform 420 showing a change in a threshold for blood flow power over time may be similar to_a waveform 410 showing a change in blood flow power over time. In addition, a threshold for blood flow power with respect to time and the blood flow power with respect to time may vary in synchronization with a change in contraction (systole) and relaxation (diastole) of the heart.

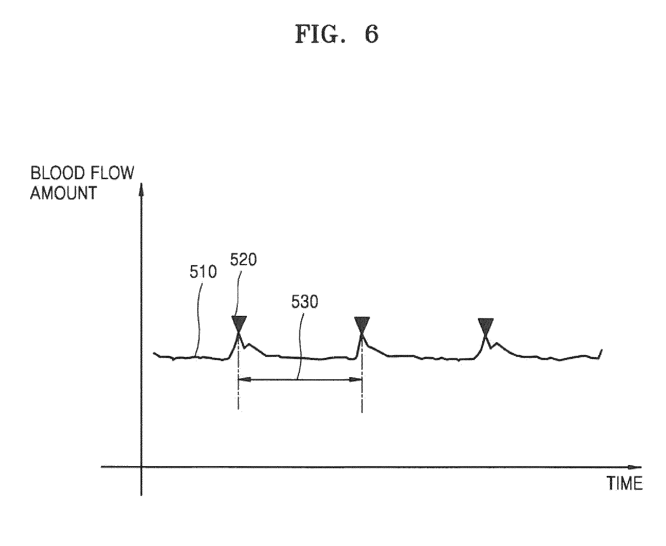

FIG. 6 is a reference diagram for explaining a method of extracting blood flow information according to an exemplary embodiment.

The blood flow information extractor 220 may analyze a change in blood flow based on color Doppler data for an ROI and obtain a waveform 510 representing a change in blood flow as shown in FIG. 6.

For example, the blood flow information extractor 220 may obtain the waveform 510 representing a change in blood flow based on a change in a value of a parameter that is set according to time. For example, the parameter may include a power threshold or power ceiling that is applied to power data of blood flow through an ROI, a velocity threshold or velocity ceiling that is applied to velocity data of the blood flow, a scale of a color flow image, and a baseline.

The blood flow information extractor 220 may extract blood flow information based on the waveform 510. The blood flow information may include at least one of a cardiac cycle duration, a heart rate, and a heartbeat landmark point for an object. The heartbeat landmark point may include a peak point 20 appearing as a heart muscle contracts and relaxes. A period 530 of the waveform 510 may represent a cardiac cycle duration. Thus, the blood flow information extractor 220 may extract a peak point (e.g., a heartbeat landmark point) appearing in the waveform 510 and calculate a time interval between adjacent peak points, thereby obtaining a cardiac cycle duration. The blood flow information extractor 220 may also calculate a heart rate based on the acquired cardiac cycle duration. The heart rate may be calculated from the reciprocal of the cardiac cycle duration.

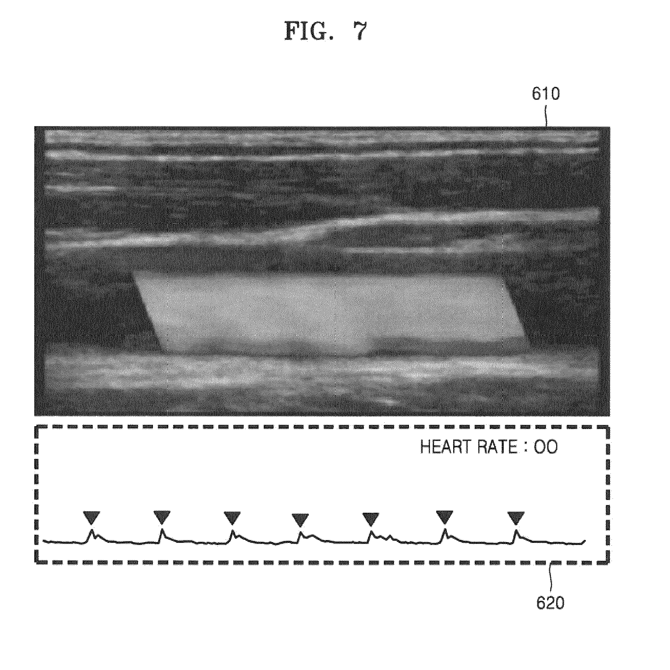

FIG. 7 is an example where a color flow image and extracted blood flow information are displayed on a display, according to an exemplary embodiment.

Referring to FIG. 7, an ultrasound image (e.g., a color flow image) and blood flow information may be respectively displayed in first and second regions 610 and 620 of the display 160 (230).

The ultrasound image displayed in the first region 610 may be an image obtained by superimposing a B-mode image and a color flow image of an ROI on each other. Furthermore, the blood flow information displayed in the second region 620 may include a waveform (graph) showing a change in blood flow over time, a heartbeat landmark point indicated in the waveform, a heart rate, a cardiac cycle duration, etc., but is not limited thereto.

For example, the heartbeat landmark point may include a peak point appearing in a waveform showing a change in blood flow as the heart contracts and relaxes. The cardiac cycle duration may be represented as a time interval between adjacent peak points in the waveform, and the heart rate may be calculated as the reciprocal of the cardiac cycle duration.

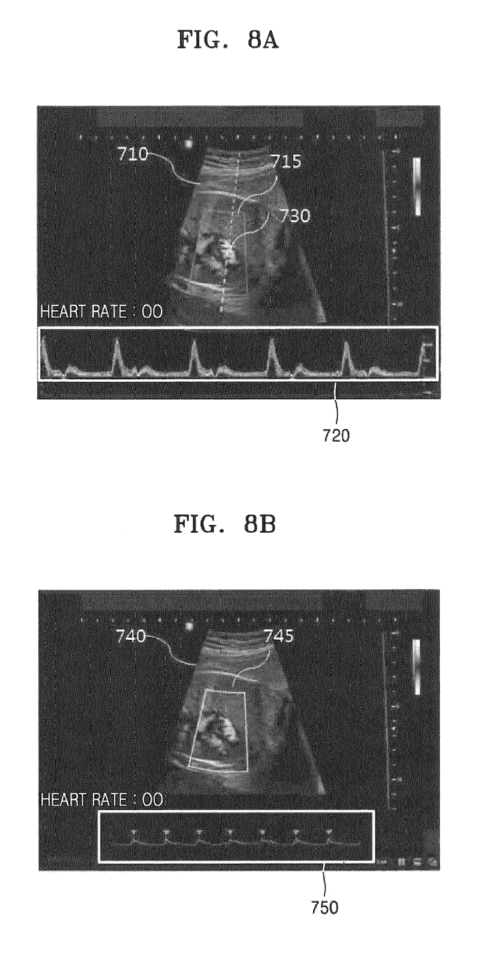

FIG. 8A is an example where an ultrasound image 710 and a spectral Doppler image 720 are displayed on a display, and FIG. 8B is an example where an ultrasound image 740 and blood flow information according to an exemplary embodiment are displayed on a display.

Referring to FIG. 8A, the ultrasound image 710 may be an image obtained by superimposing a B-mode image and a color flow image of an ROI 715 on each other. The spectral Doppler image 720 may be an image representing as a waveform Doppler data corresponding to a sample volume 730 selected from an ROI via a user input. In other words, to obtain the spectral Doppler image 720, an ultrasound diagnosis apparatus has to enter a Doppler mode to separately acquire Doppler data.

Furthermore, since the ultrasound diagnosis apparatus 100 (200) displays a waveform for Doppler data corresponding to the sample volume 730, a user needs to set the sample volume 730 in a region of an object whose waveform is to be displayed as an image. The ultrasound diagnosis apparatus 100 (200) may extract blood flow information (a heart rate, a cardiac cycle duration, a heartbeat landmark point, etc.) based on the spectral Doppler image 720 and display the extracted blood flow information.

Referring to FIG. 8B, the ultrasound image 740 may be obtained by superimposing a B-mode image and a color flow image for an ROI over each other. The ultrasound diagnosis apparatus 100 (200) may generate a color flow image of a region (ROI) selected by a color ROI box 745 and display the color flow image of the ROI.

In this case, the ultrasound diagnosis apparatus 100 (200) may analyze a change in blood flow based on color Doppler data acquired in real-time with respect to the ROI and set a parameter to be applied to the color flow image according to the change in blood flow.

Furthermore, the ultrasound diagnosis apparatus 100 (200) may acquire a waveform 750 showing a change in blood flow based on color Doppler data corresponding to an ROI and extract blood flow information based on the waveform 750. In this case, the waveform 750 may be acquired based on a change in a value of a set parameter. The ultrasound diagnosis apparatus 100 (200) may extract blood flow information by using color Doppler data acquired to generate a color flow image without having to acquire a spectral Doppler image by separately setting a sample volume as shown in FIG. 8A. Thus, convenience of use may be improved.

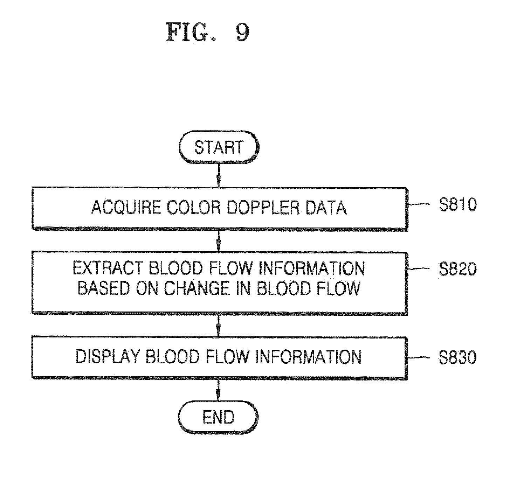

FIG. 9 is a flowchart of a method of operating the ultrasound diagnosis apparatus 100 (200) according to an exemplary embodiment.

Referring to FIG. 9, the ultrasound diagnosis apparatus 100 (200) may obtain color Doppler data for an ROI (S810).

The ultrasound diagnosis apparatus 100 (200) may acquire color Doppler data for an ROI by transmitting an ultrasound signal to the ROI and receiving an echo signal reflected from the ROI. Alternatively, the ultrasound diagnosis apparatus 100 (200) may receive color Doppler data from an external device.

For example, if the ROI is a blood vessel, the ultrasound diagnosis apparatus 100 (200) may acquire at least one of power data representing blood flow power, velocity data representing a blood flow velocity, and variance data representing blood flow variance.

The ultrasound diagnosis apparatus 100 (200) may extract blood flow information based on color Doppler data and a change in blood flow corresponding to the ROI (S820).

For example, to generate a color flow image of an ROI, the ultrasound diagnosis apparatus 100 (200) may acquire a waveform showing a change in blood flow based on a value of a parameter that is applied to color Doppler data. In this case, the parameter may include a power threshold or power ceiling that is applied to power data of blood flow, a velocity threshold or velocity ceiling that is applied to velocity data of blood flow, a scale of a color flow image, and a baseline.

A power threshold and a power ceiling may be parameters for determining a lower limit and an upper limit for power data, respectively. A velocity threshold and a velocity ceiling may be parameters for determining a lower limit and an upper limit for velocity data, respectively. A scale of a color flow image may be a parameter for determining a range of values indicated on the color flow image. A baseline may be a parameter for determining a reference value for values indicated on a color flow image.

The ultrasound diagnosis apparatus 100 (200) may analyze power data of blood flow acquired in real-time to set a power threshold or power ceiling that is applied to the power data. The ultrasound diagnosis apparatus 100 (200) may also analyze velocity data of blood velocity to set a velocity threshold or velocity ceiling that is applied to the velocity data. However, exemplary embodiments are not limited thereto, and the ultrasound diagnosis apparatus 100 (200) may set parameters necessary for generating a color flow image based on at least one selected from power data, velocity data, and variance data of blood flow.

Furthermore, each time the acquired color Doppler data changes, the ultrasound diagnosis apparatus 100 (200) may set a parameter based on the color Doppler data changed in real-time. The ultrasound diagnosis apparatus 100 (200) may then obtain a waveform showing a change in blood flow over time based on a waveform of the parameter over time.

The ultrasound diagnosis apparatus 100 (200) may extract blood flow information based on the waveform showing a change in blood flow. In this case, the blood flow information may include at least one of a cardiac cycle duration, a heart rate, and a heartbeat landmark point for an object. The heartbeat landmark point may include a peak point appearing as a heart muscle contracts and relaxes.

A period of the waveform showing a change in blood flow may represent a cardiac cycle duration. Thus, the ultrasound diagnosis apparatus 100 (200) may extract a peak point (e.g., a heartbeat landmark point) appearing in the waveform and calculate a time interval between adjacent peak points, thereby obtaining a cardiac cycle duration. The ultrasound diagnosis apparatus 100 (200) may also calculate a heart rate based on the acquired cardiac cycle duration. A heart rate may be calculated as the reciprocal of the cardiac cycle duration.

The ultrasound diagnosis apparatus 100 (200) may display the extracted blood flow information (S830).

For example, the ultrasound diagnosis apparatus 100 (200) may display a waveform showing a change in blood flow over time, a heartbeat landmark point indicated in the waveform, a heart rate, a cardiac cycle duration, etc.

Furthermore, the ultrasound diagnosis apparatus 100 (200) may display an ultrasound image. In this case, the ultrasound image may be displayed by superimposing a B-mode image of an object and a color flow image of a region of the object set as an ROI over each other. However, exemplary embodiments are not limited thereto.

The color flow image may be generated by using power data that has a value that is greater than or equal to a set power threshold or less than a set power ceiling among power data of blood flow corresponding to the ROI. The display 230 may display the generated color flow image. Alternatively, the color flow image may be generated by using velocity data that has a value that is greater than or equal to a set velocity threshold or less than a set velocity ceiling among velocity data of blood flow corresponding to the ROI.

Methods of operating an ultrasound diagnosis apparatus according to the exemplary embodiments may be embodied as a computer-readable code on a computer-readable recording medium. The computer-readable recording medium is any data storage device that can store data which can be thereafter read by a computer system. Examples of computer-readable recording media include read-only memory (ROM), random-access memory (RAM), CD-ROMs, magnetic tapes, floppy disks, optical data storage devices, transmission media such as Internet transmission media, etc.). The computer-readable recording media can also be distributed over network-coupled computer systems so that computer-readable codes are stored and executed in a distributed fashion.

While one or more exemplary embodiments have been described with reference to the figures, it will be understood by one of ordinary skill in the art that various changes in form and details may be made therein without departing from the spirit and scope of the present inventive concept as defined by the following claims. That is, all changes and modifications within the scope of the appended claims and their equivalents will be construed as being included in the present inventive concept.

## Claims

1. An ultrasound diagnosis apparatus comprising:
a data acquisition unit configured to acquire color Doppler data from a region of interest (ROI) of an object;
a blood flow information extractor configured to analyze a change in blood flow with respect to time based on the acquired color Doppler data and extract blood flow information based on the change in blood flow; and
a display configured to display the extracted blood flow information.

2. The ultrasound diagnosis apparatus of claim 1, wherein the color Doppler data comprises at least one of power data of blood flow through the ROI, velocity data of the blood flow, variance data of the blood flow, and blood flow amount data.

3. The ultrasound diagnosis apparatus of claim 1, wherein the blood flow information comprises at least one of a cardiac cycle duration, a heart rate, and a heartbeat landmark point for the object.

4. The ultrasound diagnosis apparatus of claim 3, wherein the heartbeat landmark point comprises a peak point appearing as a heart contracts and relaxes.

5. The ultrasound diagnosis apparatus of claim 1, wherein the blood flow information extractor generates a waveform showing the change in blood flow with respect to time and extracts the blood flow information based on the waveform.

6. The ultrasound diagnosis apparatus of claim 1, wherein the display displays a graph showing the change in blood flow with respect to time.

7. The ultrasound diagnosis apparatus of claim 1, wherein the blood flow information extractor sets a parameter corresponding to the ROI based on the change in blood flow and extracts the blood flow information based on a change in the parameter.

8. The ultrasound diagnosis apparatus of claim 7, wherein the parameter comprises at least one of a power threshold, a power ceiling, a velocity_threshold, a velocity ceiling, a scale, and a baseline.

9. A method of operating an ultrasound diagnosis apparatus, the method comprising:
acquiring color Doppler data from a region of interest (ROI) of an object;
analyzing a change in blood flow with respect to time based on the acquired color Doppler data and extracting blood flow information based on the change in blood flow; and
displaying the extracted blood flow information.

10. The method of claim 9, wherein the color Doppler data comprises at least one of power data of blood flow through the ROI, velocity data of the blood flow, variance data of the blood flow, and blood flow amount data.

11. The method of claim 9, wherein the blood flow information comprises at least one of a cardiac cycle duration, a heart rate, and a heartbeat landmark point for the object.

12. The method of claim 11, wherein the heartbeat landmark point comprises a peak point appearing as a heart contracts and relaxes.

13. The method of claim 9, wherein the extracting of the blood flow information comprises generating a waveform showing the change in blood flow with respect to the time and extracting the blood flow information based on the waveform.

14. The method of claim 9, wherein the displaying of the blood flow information comprises displaying a graph showing the change in blood flow with respect to the time.

15. The method of claim 9, wherein the extracting of the blood flow information comprises setting a parameter corresponding to the ROI based on the change in blood flow and extracting the blood flow information based on a change in the parameter.
